# EUROPEAN PATENT APPLICATION

(11) **EP 3 715 451 A2**
(43) Date of publication of application: **30.09.2020**
(21) Application number: 18880309.2
(22) Date of filing: 23.11.2018
(51) Int. Cl.: C12N 5/079, C07K 14/575, A61K 38/22

(54) **COMPOSITION FOR PROMOTING GOBLET CELL PROLIFERATION OR MUCIN SECRETION COMPRISING THYMOSIN BETA 4 OR DERIVATIVE THEREOF AS ACTIVE INGREDIENT**

(30) Priority: 24.11.2017 US 201762590444 P
(71) Applicant: G-Treebnt Co., Ltd., Seongnam-si, Gyeonggi-do 13554 (KR)
(72) Inventor: KANG, Sinwook, Seongnam-si Gyeonggi-do 13568 (KR); KIM, Kyoungsun, Busan 48050 (KR); YANG, Wonsuk, Namyangju-si Gyeonggi-do 12207 (KR); SUNG, Jihye, Seoul 07296 (KR)
(74) Representative: Dehns
(86) International application number: PCT/KR2018/014522
(87) International publication number: WO 2019/103523

(57) **Abstract**

The present invention relates to a composition for promoting goblet cell proliferation or mucin secretion comprising thymosin beta-4, an isoform of thymosin beta-4, an analogue thereof, or a derivative of thymosin beta-4 as an active ingredient. The composition for promoting mucin secretion comprising thymosin beta-4, an isoform thereof or derivative thereof as an active ingredient according to the present invention promotes goblet cell proliferation and increases a mucin secretion amount. In particular, the composition for promoting mucin secretion increases the expression of Muc5AC, Muc1, Muc4, and Muc16. Therefore, the composition for promoting goblet cell proliferation or mucin secretion of the present invention is considered to have an excellent effect on goblet cell or mucin-related diseases.

<110>    G-treeBNT Co., Ltd.

<120>    A composition for promoting goblet cell proliferation or
         secretion of mucin comprising thymosin beta 4 or derivative
         thereof

<130>    PCB711068GTR

<150>    US 62/590,444
<151>    2017-11-24

<160>    1

<170>    KopatentIn 2.0

<210>    1
<211>    43
<212>    PRT
<213>    Artificial Sequence

<220>
<223>    thymosine beta 4


<400>    1
Ser Asp Lys Pro Asp Met Ala Glu Ile Glu Lys Phe Asp Lys Ser Lys
  1               5                  10                  15

Leu Lys Lys Thr Glu Thr Gln Glu Lys Asn Pro Leu Pro Ser Lys Glu
             20                  25                  30

Thr Ile Glu Gln Glu Lys Gln Ala Gly Glu Ser
         35                  40

## Description

### Technical Field

The present invention relates to a composition for promoting goblet cell proliferation or mucin secretion, comprising, as an active ingredient, thymosin beta 4, thymosin beta 4 isoforms, or analogs or derivatives thereof.

### Background Art

Goblet cells are columnar epithelial cells that secrete mucin such as Muc5AC. The goblet cells are mainly distributed in the epithelium such as in small intestine, large intestine, bronchus conjunctiva, and the like. The goblet cells secrete mucin to moisten the mucosal surface, thereby protecting tissues from mechanical friction or chemical attack.

On the other hand, mucin is a glycoprotein secreted from epithelial tissue such as in gastrointestinal tract, lung, kidney, ovary, breast, eye, nose, pancreas, and the like. Under a normal physiological condition, mucin serves to protect epithelial tissue. However, in a case where mucin is poorly secreted from epithelial tissue, diseases such as gastritis, gastric ulcer, enteritis, ulcerative colitis, constipation, and the like can be caused. In addition, in a case where mucin is poorly secreted from the mucosa of organs such as eyes and nose, external harmful substances may enter the body and may cause a disease such as keratitis, dry eye, rhinitis, bronchitis, pneumonia, and the like.

Meanwhile, thymosin beta 4 is a protein consisting of 41 to 43 amino acids and having an isoelectric point of 5.1, which has been first found in the thymus in 1981. Thymosin beta 4 was first identified as an actin-sequestering molecule in animal cells by Riva et al. in 1991. Subsequently, it was found that thymosin beta 4 is also involved in immunoregulation and neuroendocrine.

### Disclosure of Invention

### Technical Problem

Accordingly, the present inventors have conducted studies on compositions that can effectively promote mucin secretion. As a result, the present inventors have identified that thymosin beta 4 not only effectively proliferates goblet cells, but also promotes mucin secretion; and thus have completed the present invention.

### Solution to Problem

In an aspect of the present invention, there is provided a composition for promoting goblet cell proliferation or mucin secretion comprising, as an active ingredient, thymosin beta 4, thymosin beta 4 isoforms, or analogs or derivatives thereof.

In another aspect of the present invention, there is provided a pharmaceutical composition for preventing or treating goblet cell-related or mucin-related diseases comprising, as an active ingredient, thymosin beta 4, thymosin beta 4 isoforms or derivatives thereof.

In yet another aspect of the present invention, there is provided a method for treating goblet cell-related or mucin-related diseases, comprising administering the composition to a subject.

In still yet another aspect of the present invention, there is provided a use of the composition for promoting goblet cell proliferation or mucin secretion.

In still yet another aspect of the present invention, there is provided a use of the composition for manufacturing of a medicament for promoting goblet cell proliferation or mucin secretion.

In still yet another aspect of the present invention, there is provided a use of the pharmaceutical composition for preventing or treating goblet cell-related or mucin-related diseases.

In still yet another aspect of the present invention, there is provided a use of the pharmaceutical composition for manufacturing of a medicament for preventing or treating goblet cell-related or mucin-related diseases.

### Advantageous Effects of Invention

A composition for promoting mucin secretion which comprises, as an active ingredient, thymosin beta 4, thymosin beta 4 isoforms, or analogs or derivatives thereof of the present invention promotes goblet cell proliferation and increases mucin secretion. In particular, the composition for promoting mucin secretion increases expression of Muc5AC, Muc1, Muc4, and Muc16. Therefore, it is believed that a composition for promoting mucin secretion of the present invention exhibits an excellent effect on goblet cell-related or mucin-related diseases.

### Brief Description of Drawings

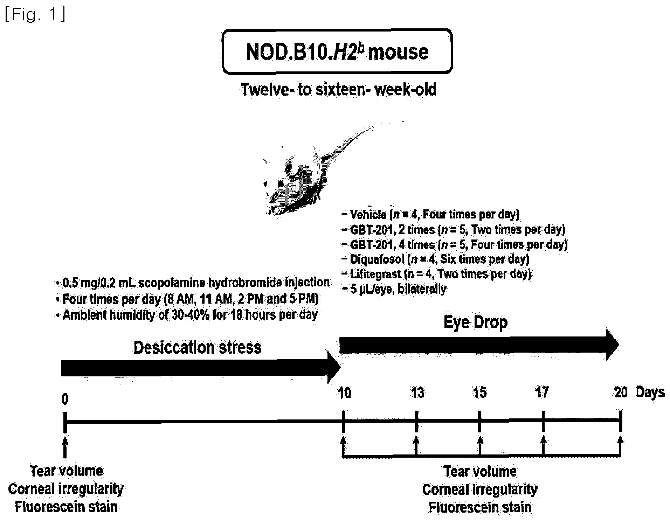

Fig. 1 illustrates a schematic diagram of an animal experiment using dry eye-induced mice, intended to identify an effect of thymosin beta 4 on promotion of goblet cell proliferation and mucin secretion.

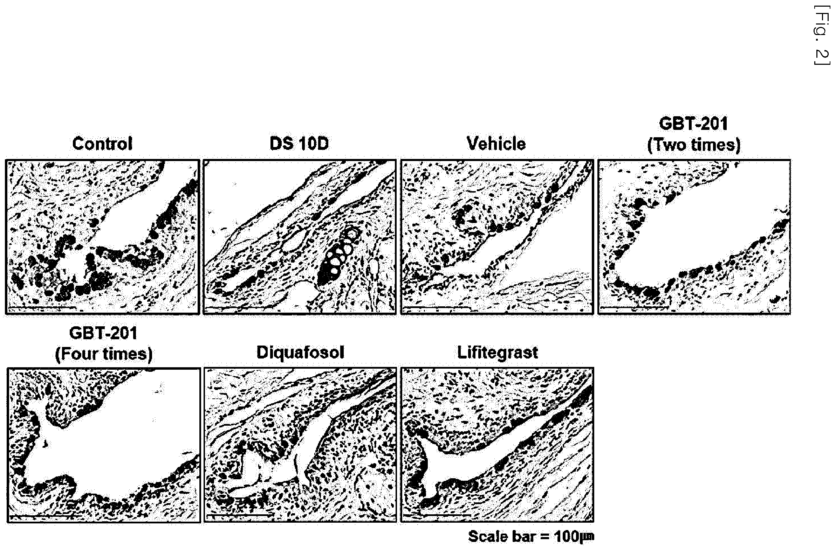

Fig. 2 illustrates photographs taken after collecting the eyeballs of normal mice and dry eye-induced mice that have been subjected to eye drop instillation with test substances, and staining goblet cells in tissues, so as to identify an effect of thymosin beta 4 on promotion of goblet cell proliferation.

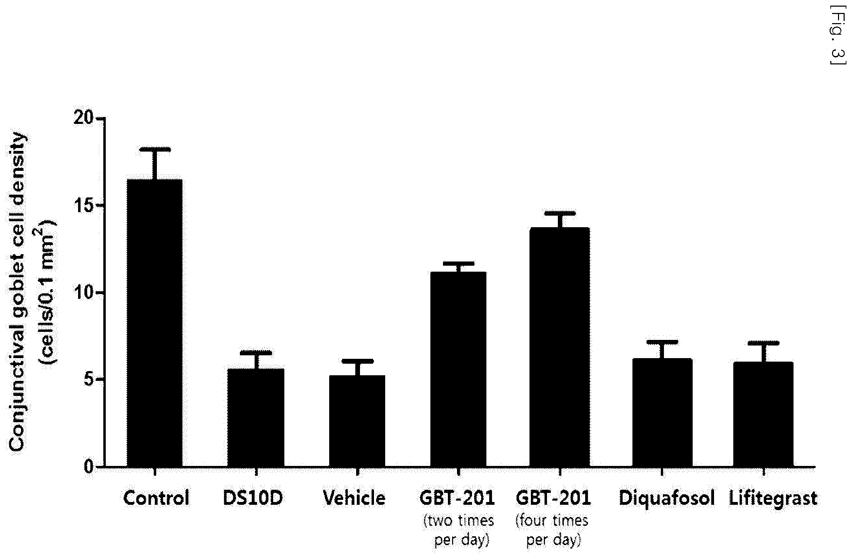

Fig. 3 illustrates results obtained by collecting the eyeballs of normal mice and dry eye-induced mice that have been subjected to eye drop instillation with test substances, staining goblet cells in tissues, and then quantifying the stained areas, so as to identify an effect of thymosin beta 4 on promotion of goblet cell proliferation.

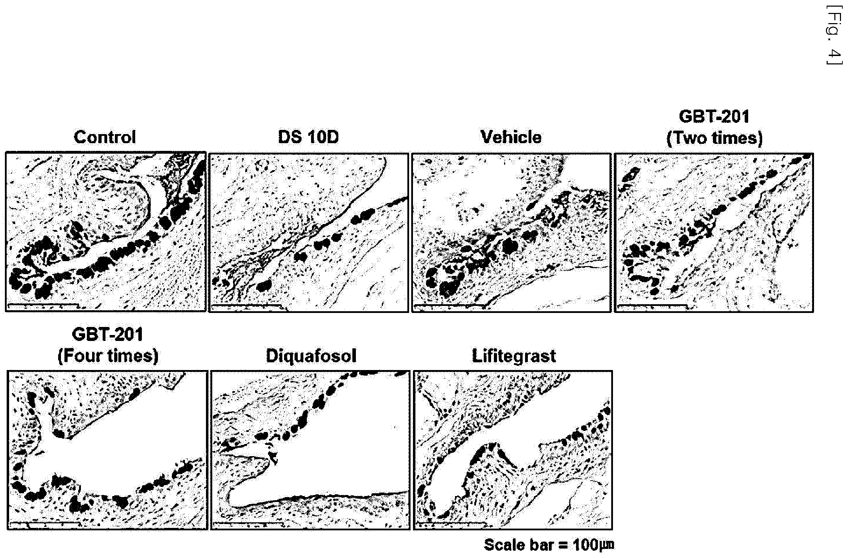

Fig. 4 illustrates photographs taken after collecting the eyeballs of normal mice and dry eye-induced mice that have been subjected to eye drop instillation with test substances, and staining mucin in tissues, so as to identify an effect of thymosin beta 4 on promotion of mucin secretion.

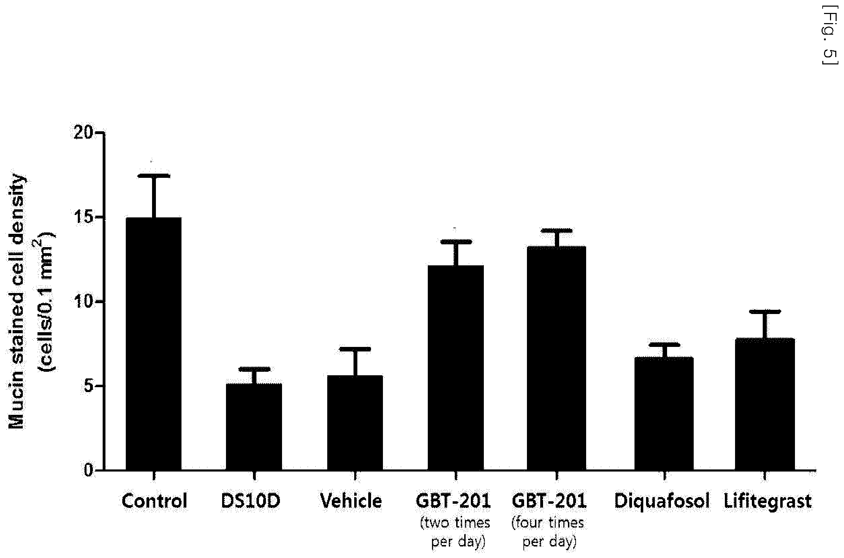

Fig. 5 illustrates results obtained by collecting the eyeballs of normal mice and dry eye-induced mice that have been subjected to eye drop instillation with test substances, staining mucin in tissues, and then quantifying the stained areas, so as to identify an effect of thymosin beta 4 on promotion of mucin secretion.

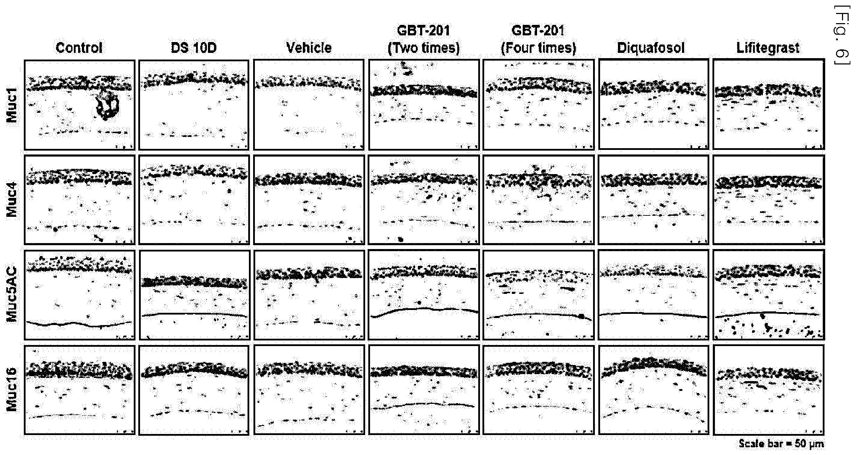

FIG. 6 illustrates photographs taken after collecting the eyeballs of normal mice and dry eye-induced mice that have been subjected to eye drop instillation with test substances, and staining, with immunofluorescence, Muc1, Muc4, Mucl6, and Muc5AC which are expressed in the cornea, so as to identify an effect of thymosin beta 4 on increase in expression of Muc1, Muc4, Muc16, and Muc5AC.

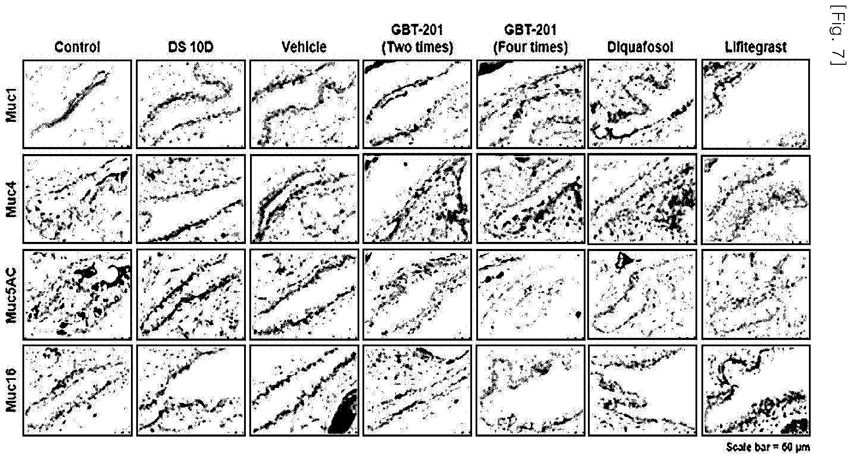

Fig. 7 illustrates photographs taken after collecting the eyeballs of normal mice and dry eye-induced mice that have been subjected to eye drop instillation with test substances, and staining, with immunofluorescence, Muc1, Muc4, Mucl6, and Muc5AC which are expressed in the conjunctiva, so as to identify an effect of thymosin beta 4 on increase in expression of Muc1, Muc4, Muc16, and Muc5AC.

### Best Mode for Carrying out the Invention

Hereinafter, the present invention will be described in more detail.

In an aspect of the present invention, there is provided a composition for promoting goblet cell proliferation or mucin secretion, comprising, as an active ingredient, thymosin beta 4, thymosin beta 4 isoforms, or analogs or derivatives thereof.

As used herein, the term "thymosin beta 4", which is a protein also called Tβ4, refers to a polypeptide of 4.9 kDa consisting of 43 amino acids which was first isolated from the thymus and has been identified in various tissues. Thymosin beta 4 is a protein upregulated during endothelial cell migration and differentiation *in vitro.* Many isoforms of thymosin beta 4 have been identified and these isoforms have at least about 70%, about 75%, or about 80% identity to the known amino acid sequence of thymosin beta 4. Thymosin beta 4 can be a protein having the amino acid sequence of SEQ ID NO: 1.

A derivative of thymosin beta 4 may have a mutated N-terminus or C-terminus of thymosin beta 4. The derivative of thymosin beta 4 may have partial truncation in the N-terminus and/or the C-terminus of thymosin beta 4 or addition of one or more amino acids thereto, as long as activity of thymosin beta 4 is maintained. Specifically, the derivative of thymosin beta 4 may have addition of 1 or 2 amino acids to the N-terminus of thymosin beta 4. In addition, the derivative of thymosin beta 4 may have addition of 1 or 2 amino acids to the C-terminus of thymosin beta 4.

Here, the amino acid can be, but is not limited to, any one or combination selected from the group consisting of glycine, alanine, arginine, aspartate, cysteine, glutamate, glutamine, histidine, proline, serine, tyrosine, isoleucine, leucine, lysine, tryptophan, valine, methionine, phenylalanine, asparagine, and threonine.

The derivative of thymosin beta 4 may have truncation of 1 to 3 amino acids in the N-terminus of thymosin beta 4. In addition, the derivative of thymosin beta 4 may have truncation of 1 to 3 amino acids in the C-terminus of thymosin beta 4.

The concentration of thymosin beta 4, thymosin beta 4 isoforms, or analogs or derivatives thereof in the composition for promoting goblet cell proliferation or mucin secretion can be 0.01% (w/v) to 1.0% (w/v), preferably 0.02% (w/v) to 0.5% (w/v).

As used herein, the term "goblet cells" refers to mucus-secreting cells present in the mucosal epithelial lining. The goblet cells are columnar cells that secrete mucin such as Muc5AC. In addition, the goblet cells are mainly distributed in the epithelium such as in small intestine, large intestine, bronchus, and conjunctiva and the like. The goblet cells secrete mucin to moisten the mucosal surface, thereby protecting tissue from mechanical friction or chemical attack.

As described above, when dysfunction occurs in goblet cells that serve to protect the mucosa of each tissue, mucin secretion in an epithelial tissue, such as in gastrointestinal tract, lung, kidney, ovary, pancreas, eye, and nose, is decreased and thereby may cause a disease. The disease caused by dysfunction in goblet cells can be, but is not limited to, gastritis, gastric ulcer, enteritis, ulcerative colitis, or dry eye.

The composition can be formulated and used according to conventional methods. Suitable formulations include, but are not limited to, hard or soft capsules, solutions, suspensions, emulsions, injections, suppositories, ophthalmic formulations, and the like.

The composition can be prepared into a suitable formulation using an inert organic or inorganic carrier. That is, when the formulation is a hard capsule, the composition may contain lactose, sucrose, starch or derivatives thereof, or talc, calcium carbonate, gelatin, stearic acid or salts thereof. In addition, when the formulation is a soft capsule, the composition can contain vegetable oil, wax, fat, a semi-solid, or liquid polyol. Furthermore, when the formulation is in the form of a solution or syrup, the composition can contain water, polyol, glycerol, and/or vegetable oil, and the like.

In addition to the carrier, the composition can further contain a preservative, a stabilizer, a wetting agent, an emulsifying agent, a solubilizing agent, a sweetener, a colorant, an osmotic pressure regulator, an antioxidant, and the like.

When the formulation of the composition is an ophthalmic formulation, the composition can further contain acetic acid and citric acid, salts thereof, or hydrates of the salts. In addition, the composition can additionally contain sodium citrate hydrate or sodium acetate hydrate.

Citric acid is a compound having the formula C₆H₈O₇. In addition, citric acid can be used in the form of citrate. Citrate is a derivative of citric acid; and for example, citrate can be sodium citrate or sodium citrate hydrate. Citric acid or salts thereof is usually used as a buffer for minimizing pH change. However, citric acid or salts thereof used in the present invention should be used in a larger amount than that normally used. Here, citric acid or salts thereof can be contained in an amount of 0.01% (w/v) to 0.5% (w/v) in the total composition. In addition, citric acid or salts thereof can be contained in an amount of 0.05% (w/v) to 0.25% (w/v) in the total composition, preferably in an amount of 0.1% to 0.3% in the total composition.

Acetic acid is a weak acid having the formula CH₃COOH. In addition, acetic acid can be used in the form of acetate. For example, the acetate can be sodium acetate hydrate. Here, acetic acid or salts thereof can be contained in an amount of 0.01% (w/v) to 1.5% (w/v) in the total composition. In addition, acetic acid or salts thereof can be contained in an amount of 0.1% (w/v) to 0.8% (w/v) in the total composition, preferably in an amount of 0.2% to 0.5% in the total composition.

The composition can further contain sodium chloride, potassium chloride, calcium chloride dihydrate, and magnesium chloride hexahydrate.

In addition, the concentration of sodium chloride can be 0.1% (w/v) to 1.2% (w/v), and can be 0.3% (w/v) to 1.0% (w/v). Preferably, the concentration of sodium chloride can be 0.5% (w/v) to 0.7% (w/v). In addition, the concentration of potassium chloride can be 0.01% (w/v) to 0.15% (w/v), and can be 0.03% (w/v) to 0.12% (w/v). Preferably, the concentration of potassium chloride can be 0.05% (w/v) to 0.09% (w/v). In addition, the concentration of calcium chloride dihydrate can be 0.01% (w/v) to 0.12% (w/v), and can be 0.03% (w/v) to 0.09% (w/v). Preferably, the concentration of calcium chloride dihydrate can be 0.03% (w/v) to 0.06% (w/v). In addition, the concentration of magnesium chloride hexahydrate can be 0.01% (w/v) to 0.12% (w/v), and can preferably be 0.01% (w/v) to 0.05% (w/v).

In addition, the composition can further contain hydrochloric acid or sodium hydroxide. Hydrochloric acid or sodium hydroxide can be added in an appropriate amount to adjust the pH of the composition. In addition, the pH of the composition can be 6.5 to 7.5, and can be 6.8 to 7.2. Preferably, the pH of the composition can be 7.0.

The present inventors conducted experiments using dry eye-induced mice in order to identify effects of thymosin beta 4 on promotion of goblet cell proliferation and mucin secretion and on treatment of mucin-related diseases.

Specifically, the dry eye-induced mice were subjected to eye drop instillation with each of thymosin beta 4 and other test drugs for 10 days. After 10 days, each group of dry eye-induced mice was sacrificed, the eyeballs were collected, and tissue immunostaining and molecular biological analysis were performed (Fig. 1). As a result, it was identified that the number of goblet cells was increased in the dry eye-induced mice that had been subjected to eye drop instillation with thymosin beta 4 (Figs. 2 and 3). In addition, it was identified that the amount of mucin secreted was increased in the dry eye-induced mice that had been subjected to eye drop instillation with thymosin beta 4 (Figs. 4 and 5). Furthermore, it was identified that expression levels of Muc1, Muc4, Muc16, and Muc5AC were increased in the dry eye-induced mice that had been subjected to eye drop instillation with thymosin beta 4 (Figs. 6 and 7).

From these results, it was identified that thymosin beta 4 proliferated goblet cell and promoted mucin secretion and thus can be used for preventing or treating mucin-related diseases.

In another aspect of the present invention, there is provided a pharmaceutical composition for preventing or treating goblet cell-related or mucin-related diseases, comprising, as an active ingredient, thymosin beta 4, thymosin beta 4 isoforms, or analogs or derivatives thereof.

The thymosin beta 4, thymosin beta 4 isoforms, analogs, and derivatives thereof are as described above.

As used herein, the term "goblet cell-related or mucin-related diseases" refers to diseases caused by decrease of mucin secretion from epithelial tissue such as in gastrointestinal tract, lung, kidney, ovary, pancreas, eye, nose and the like. The goblet cell-related or mucin-related diseases can be, but is not limited to, gastritis, gastric ulcer, enteritis, ulcerative colitis, or dry eye.

The pharmaceutical composition can further contain a pharmaceutically acceptable carrier. The term "pharmaceutically acceptable" means that the carrier does not significantly irritate an organism and does not interfere with biological activity and properties of the active substance to be administered.

The carrier can be natural or synthetic. Formulations can be prepared by using various carriers such as diluents or excipients, including fillers, extenders, binders, wetting agents, disintegrants, and surfactants, depending on the formulation.

For example, solid formulations for oral administration include capsules and the like. Such solid formulations can be prepared by mixing one or more compounds with at least one excipient, for example, starch, calcium carbonate, sucrose or lactose, gelatin, and the like. In addition, besides simple excipients, lubricants such as magnesium stearate and talc may also be used. Liquid formulations for oral administration include suspensions, solutions, emulsions, syrups, and the like. The liquid formulations can contain various excipients such as wetting agents, sweetening agents, fragrances, and preservatives, in addition to water and liquid paraffin which are diluents.

Formulations for parenteral administration include sterile aqueous solutions, non-aqueous solutions, suspensions, emulsions, freeze-dried formulations, and suppositories. For the non-aqueous solutions and the suspensions, propylene glycol, polyethylene glycol, vegetable oil such as olive oil, injectable ester such as ethyl oleate, and the like can be used. As bases for the suppositories, Witepsol, macrogol, Tween 61, cacao fat, laurin fat, glycerogelatin, and the like can be used. The pharmaceutical compositions can be formulated and used according to respective conventional methods. Suitable formulations include, but are not limited to, hard or soft capsules, solutions, suspensions, emulsions, injections, suppositories, ophthalmic formulations, and the like.

In addition to the carrier, the pharmaceutical composition can further contain a preservative, a stabilizer, a wetting agent, an emulsifying agent, a solubilizing agent, a sweetener, a colorant, an osmotic pressure regulator, an antioxidant, and the like.

The concentration of thymosin beta 4, thymosin beta 4 isoforms, or analogs or derivatives thereof in the pharmaceutical composition can be 0.01% (w/v) to 1.0% (w/v), preferably 0.02% (w/v) to 0.5% (w/v).

When the pharmaceutical composition is in the form of an ophthalmic formulation, the composition can further contain acetic acid and citric acid, salts thereof, or hydrates of the salts. In addition, the composition can additionally contain sodium citrate hydrate or sodium acetate hydrate.

Citric acid is a compound having the formula C₆H₈O₇. In addition, citric acid can be used in the form of citrate. Citrate is a derivative of citric acid; and for example, citrate can be sodium citrate or sodium citrate hydrate. Citric acid or salts thereof is usually used as a buffer for minimizing pH change. However, citric acid or salts thereof used in the present invention should be used in a larger amount than that normally used. Here, citric acid or salts thereof can be contained in an amount of 0.01% (w/v) to 0.5% (w/v) in the total composition. In addition, citric acid or salts thereof can be contained in an amount of 0.05% (w/v) to 0.25% (w/v) in the total composition, preferably in an amount of 0.1% to 0.3% in the total composition.

Acetic acid is a weak acid having the formula CH₃COOH. In addition, acetic acid can be used in the form of acetate. For example, the acetate can be sodium acetate hydrate. Here, acetic acid or salts thereof can be contained in an amount of 0.01% (w/v) to 1.5% (w/v) in the total composition. In addition, acetic acid or salts thereof can be contained in an amount of 0.1% (w/v) to 0.8% (w/v) in the total composition, preferably in an amount of 0.2% to 0.5% in the total composition.

When the formulation of the pharmaceutical composition is an ophthalmic formulation, the composition can further contain sodium chloride, potassium chloride, calcium chloride dihydrate, and magnesium chloride hexahydrate.

In addition, the concentration of sodium chloride can be 0.1% (w/v) to 1.2% (w/v), and can be 0.3% (w/v) to 1.0% (w/v). Preferably, the concentration of sodium chloride can be 0.5% (w/v) to 0.7% (w/v). In addition, the concentration of potassium chloride can be 0.01% (w/v) to 0.15% (w/v), and can be 0.03% (w/v) to 0.12% (w/v). Preferably, the concentration of potassium chloride can be 0.05% (w/v) to 0.09% (w/v). In addition, the concentration of calcium chloride dihydrate can be 0.01% (w/v) to 0.12% (w/v), and can be 0.03% (w/v) to 0.09% (w/v). Preferably, the concentration of calcium chloride dihydrate can be 0.03% (w/v) to 0.06% (w/v). In addition, the concentration of magnesium chloride hexahydrate can be 0.01% (w/v) to 0.12% (w/v), and can preferably be 0.01% (w/v) to 0.05% (w/v).

In addition, the pharmaceutical composition can additionally contain hydrochloric acid or sodium hydroxide. Hydrochloric acid or sodium hydroxide can be added in an appropriate amount to adjust the pH in the composition. In addition, the pH of the composition can be 6.5 to 7.5, and can be 6.8 to 7.2. Preferably, the pH of the composition can be 7.0.

In addition, when the pharmaceutical composition is in the form of an ophthalmic formulation, the composition can be formulated by being mixed with ophthalmically acceptable non-toxic excipients or carriers. For example, those as mentioned below, in particular, carriers, stabilizers, solubilizers, buffer substrates, preservatives, tonicity agents, thickeners, and other excipients can be used. In addition, the solution can be adjusted to a desired pH and used.

The carriers that can be used according to the present invention are typically suitable for topical or systemic administration, and examples thereof include water, a mixture of water and water-miscible solvents such as C₁-C₇ alkanols, vegetable oils or mineral oils such as 0.5 to 5 wt% of hydroxyethyl cellulose, ethyl oleate, carboxymethyl cellulose, polyvinyl pyrrolidone, and other non-toxic water-soluble polymers for ophthalmic use, for example, cellulose derivatives such as methyl cellulose, alkali metal salts of carboxymethyl cellulose, hydroxymethyl cellulose, hydroxyethyl cellulose, methyl hydroxypropyl cellulose, and hydroxypropyl cellulose, acrylates or methacrylates such as salts of polyacrylic acid or ethyl acrylate, polyacrylamides, natural products such as gelatin, alginates, pectins, tragacanth, karaya gum, xanthan gum, carrageenan, agar, acacia, starch derivatives such as starch acetate and hydroxypropyl starch, and other synthetic products, for example, polyvinyl alcohol, polyvinyl pyrrolidone, polyvinyl methyl ether, polyethylene oxide, preferably, cross-linked polyacrylic acid such as neutral carbopol, or mixtures of these polymers. Preferred examples of the carriers include water, cellulose derivatives, for example, methyl cellulose, alkali metal salts of carboxymethyl cellulose, hydroxymethyl cellulose, hydroxyethyl cellulose, methyl hydroxypropyl cellulose and hydroxypropyl cellulose, neutral carbopol, or mixtures thereof.

Examples of the stabilizers include tyloxapol, fatty-acid glycerol poly-lower alkylene glycol esters, fatty-acid poly-lower alkylene glycol esters, polyethylene glycols, glycerol ethers, or mixtures of these compounds. The stabilizer is typically added in an amount sufficient to dissolve an active ingredient.

Examples of the buffers include borate, hydrogen carbonate/carbonate, gluconate, phosphate, propionate, and tromethamine (TRIS) buffers. Tromethamine and borate buffers are preferred. The buffer substrate is added, for example, in an amount to ensure and maintain a physiologically acceptable pH range. The pH range is typically pH 5 to 9, preferably pH 6 to 8.2, and more preferably pH 6.8 to 8.1.

Examples of the preservatives include quaternary ammonium salts such as cetrimide, benzalkonium chloride, or benzoxonium chloride; alkyl-mercury salts of thiosalicylic acid such as thimerosal, phenylmercuric nitrate, phenylmercuric acetate, or phenylmercuric borate, parabens such as phenylparaben or propylparaben, alcohols such as chlorobutanol, benzyl alcohol, or phenyl ethanol, guanidine derivatives such as chlorohexidine or polyhexamethylene biguanide, or sorbic acid. Preferred examples of the preservatives include cetrimide, benzalkonium chloride, benzoxonium chloride, and parabens. The preservative can be added in a sufficient and adequate amount to prevent secondary contamination caused by bacteria and fungi during use.

Among those mentioned herein, the tonicity agent is used to adjust the tonicity of a target product to physiological isotonicity (for example, 0.9% saline). For example, sodium chloride, potassium chloride, calcium chloride, dextrose and/or mannitol can be added to the composition comprising thymosin beta 4 of the present invention. An amount of the tonicity agent varies depending on types of specific agents to be added. In general, in particular compositions of the present invention, the tonicity agent can be added such that the final composition has an ophthalmically acceptable osmolarity of preferably 150 mOsm to 450 mOsm and most preferably 250 mOsm to 350 mOsm. Preferred examples of the tonicity agent include sodium salts and potassium salts, in particular, sodium chloride and potassium chloride. The most preferred tonicity agent is sodium chloride.

In addition, in order to maintain a proper viscosity in the ophthalmic formulation, the following agents can be used, but the present invention is not be limited thereto: (a) monomeric polyols such as tyloxapol, glycerol, propylene glycol, ethylene glycol; (b) polymeric polyols such as polyethylene glycol (for example, PEG 300, PEG 400); (c) cellulose derivatives (cellulose-based polymers) such as hydroxyethyl cellulose, hypromellose, hydroxypropyl methyl cellulose, methyl cellulose, sodium carboxymethyl cellulose, hydroxypropyl cellulose; (d) dextrans such as dextran 70; (e) water-soluble proteins such as gelatin; (f) vinyl polymers such as polyvinyl alcohol, polyvinyl pyrrolidine; (g) other polyols such as polysorbate 80, povidone; (h) carbomers such as carbomer 934P, carbomer 941, carbomer 940, and carbomer 974P; and (i) polysaccharides/glycosaminoglycans such as hyaluronan (hyaluronic acid/hyaluronate), chondroitin sulfate. In addition, at least one viscosity enhancer can be added to the composition of the present invention so that a viscosity of the carrier (vehicle) is increased.

The amounts and types of excipients added can vary depending on specific requirements. The excipients can be usually used in a range of about 0.0001 wt% to about 90 wt%, and can be used within a range commonly used by those skilled in the ophthalmology field. In addition, the ophthalmic formulations can have a pH range of 3.5 to 9, preferably 4.5 to 8, and most preferably 5.5 to 7.8.

In yet another aspect of the present invention, there is provided a method for treating goblet cell-related or mucin-related diseases, comprising administering the pharmaceutical composition to an individual.

Specifically, the method of treating goblet cell-related or mucin-related diseases can be a treatment method comprising bringing, into contact with a tissue, an effective amount of a pharmaceutical composition which contains, as an active ingredient, thymosin beta 4 or derivatives thereof. Examples of direct administration include direct application of a solution, lotion, salve, gel, cream, paste, spray, suspension, dispersion, hydrogel, ointment, oil, or foams, which contains a peptide agent as disclosed herein, to come into contact with the tissue.

The goblet cell-related or mucin-related diseases are as described above.

In addition, the composition can further contain citric acid or salts thereof. In addition, the composition can further contain citric acid and acetic acid, or salts thereof. Here, thymosin beta 4 and citric acid or salts thereof can be administered sequentially or in combination, and can be administered in an appropriate amount with divided doses several times a day. The simultaneous combined administration of thymosin beta 4 and citric acid or salts thereof is most preferred.

Thymosin beta 4 in the composition can be contained in an amount of 0.01% (w/v) to 1.0% (w/v) or 0.02% (w/v) to 0.5% (w/v) based on the total amount of the composition, indicating that thymosin beta 4 can be administered at a total daily dose of 0.08 ml to 2.0 ml. Thymosin beta 4 can be administered once or several times a day, preferably 2 to 5 times a day. In addition, citric acid or salts thereof can be contained in an amount of 0.01% (w/v) to 0.5% (w/v) based on the total amount of the composition; or can be contained in an amount of 0.05% (w/v) to 0.25% (w/v), preferably 0.1% (w/v) to 0.3 (w/v), based on the total amount of the composition. Alternatively, citric acid or salts thereof can be administered at a total daily dose of 0.1 ml to 4.0 ml. Citric acid or salts thereof can be administered once or several times a day, preferably 2 to 5 times a day.

In addition, acetic acid or salts thereof can be administered sequentially or in combination with thymosin beta 4 and citric acid or salts thereof. Preferably, acetic acid or salts thereof can be administered simultaneously with thymosin beta 4 and citric acid or salts thereof. Here, acetic acid or salts thereof can be contained in an amount of 0.01% (w/v) to 1.5% (w/v) based on the total amount of the composition; or can be contained in an amount of 0.1% (w/v) to 0.8% (w/v), preferably 0.2% (w/v) to 0.5 (w/v), based on the total amount of the composition. Alternatively, acetic acid or salts thereof can be administered at a total daily dose of 0.15 ml to 6.0 ml. Acetic acid or salts thereof can be administered once or several times a day, preferably 2 to 5 times a day.

Examples of routes of administration for the composition include, but are not limited to, oral administration and parenteral administration such as intravenous, intradermal, subcutaneous, intranasal (for example, inhalation), transdermal (for example, topical), mucosal, and rectal administration.

In still yet another aspect of the present invention, there is provided a use of the composition of the present invention for promoting goblet cell proliferation or mucin secretion.

In still yet another aspect of the present invention, there is provided a use of the composition of the present invention for manufacturing of a medicament for promoting goblet cell proliferation or mucin secretion.

In still yet another aspect of the present invention, there is provided a use of the pharmaceutical composition for preventing or treating goblet cell-related or mucin-related diseases.

In still yet another aspect of the present invention, there is provided a use of the pharmaceutical composition for manufacturing of a medicament for preventing or treating goblet cell-related or mucin-related diseases.

### Mode for the Invention

Hereinafter, the present invention will be described in detail by way of examples and the like to help understand the present invention. However, according to the present invention, the examples can be modified in various other forms, and the scope of the present invention should not be interpreted as being limited to the following examples.

### Example 1. Preparation of composition containing thymosin beta 4 (GBT-201)

Each weighed reagent was added in sterile water for injection so that they have the amounts as shown in Table 1 below, and mixing was performed until the respective reagents are completely dissolved. Then, thymosin beta 4 (Bachem, USA, SEQ ID NO: 1) to a concentration of 1 mg/ml was added in the mixed solution, and mixing was performed until it was completely dissolved. For the solution obtained after the above mixing process, acidity thereof was adjusted to 7.0 using sodium hydroxide and hydrochloric acid. Thereafter, the solution having undergone the above adjustment process was filtered through a 0.2 µm filter. Then, a low-density polyethylene container was filled with the mixed solution having undergone the above filtration process, and sealed.

**[Table 1]**

| Reagent | Amount added (mg/ml) | % (w/v) | Function |
|---|---|---|---|
| Thymosin beta 4 | 1 | 0.1 | Active ingredient |
| Sodium chloride | 6.4 | 0.64 | Isotonic agent |
| Calcium chloride dihydrate | 0.3 | 0.03 | Electrolyte |
| Citric acid | 1.0 | 0.10 | Buffer |
| Hydrochloric acid | Added as needed to adjust pH to 7.0 | | pH adjusting agent |
| Sodium hydroxide | Added as needed to adjust pH to 7.0 | | pH adjusting agent |
| Sterile water for injection | Q.S. to 100 % (w/v) | | Solvent |

### Experimental Example 1. Preparation of dry eye-induced mice and eye drop instillation with drug

12-week-old or older NOD.B10-H2b mice were kept in a chamber with a humidity of 40% or lower for 10 days. Scopolamine hydrobromide (Sigma-Aldrich, SLBR8568V) at a concentration of 0.5 mg/0.2 ml was injected subcutaneously into the thigh 4 times (9 am, 12 pm, 3 pm, and 6 pm) daily while the mice were kept in the chamber. After 10 days, tear secretion volume and corneal smoothness score were measured, and only those mice with a tear secretion volume of 0.06 µl or lower and a corneal smoothness score of 2 or lower were selected. The selected mice were grouped as shown in Table 2 below.

**[Table 2]**

| Group | Normal control | DS D10 control | Vehicle | GBT-201, twice | GBT-201, 4 times | Diquas, 6 times | Xiidra, twice |
|---|---|---|---|---|---|---|---|
| Number | 4 | 4 | 4 | 5 | 5 | 4 | 4 |

After grouping as shown in Table 2, each group of mice was subjected to eye drop instillation with the corresponding test drug for 10 days. For each test drug, eye drop instillation was performed as follows: Vehicle, 4 times a day; Diquas (Santen Pharmaceutical Co., Ltd., Japan), 6 times a day; Xiidra (Shire plc, USA), twice a day; and GBT-201, twice or 4 times a day. After 10 days, each group of mice was sacrificed, the eyeballs were collected, and tissue immunostaining and molecular biological analysis were performed (Fig. 1).

### Experimental Example 2. Identification of effect of thymosin beta 4 on promotion of goblet cell proliferation

In order to identify whether thymosin beta 4 promotes proliferation of goblet cells, which are cells that secrete mucin in the conjunctiva, the eyeballs of the mice sacrificed in Experimental Example 1 were collected and assessed through periodicacid-Schiff (PAS) staining. For the sections of each group, 0.1 mm² region of the cornea or inferior fornices of the conjunctiva was evaluated.

Specifically, the eyeballs of the mice sacrificed in Experimental Example 1 were collected, and the eyes and adnexa, which had been fixed in formalin for 3 days, were cut into 6 µm sections using a microtome. The sections of eyes and adnexa cut in 6 µm thickness were stained using a PAS kit (Merck Chemicals International, USA). If necessary, each of the sections was deparaffinized and hydrated with distilled water. The hydrated section was washed with distilled water, and then treated with periodic acid. Thereafter, the resulting section was oxidized by being treated with aldehyde, and stained red-purple by being reacted with a Schiff reagent for 10 minutes. The section was counter-stained with hematoxylin, and then subjected to dehydration and clarification processes. Mounting thereof was performed using a mounting medium. For the mounted slide, photographing and tissue analysis were performed with a virtual microscope (NanoZoomer 2.0 RS, Hamamatsu Photonics K.K., Japan).

As a result, when the normal control group and the DS D10 control group were compared, it was visually identified that the number of goblet cells was decreased in the DS D10 control group as compared with the normal control group. On the other hand, as for the group that had been subjected to eye drop instillation with GBT-201, Diquas, or Xiidra, it was visually identified that the number of goblet cells was increased as compared with the DS D10 control group (Fig. 2).

Specifically, when the stained areas were quantified, the following results were observed. The number of goblet cells in the DS D10 control group was decreased by about 65.9% as compared with the number of goblet cells in the normal control group (16.476 ± 1.722/0.1 mm² vs 5.619 ± 0.918 cells/0.1 mm²). On the other hand, the number of goblet cells in the GBT-201 group was increased about 2-fold to 2.4-fold as compared with the number of goblet cells in the DS D10 control group (11.143 ± 0.495 cells/0.1 mm², 13.619 ± 0.918 cells/0.1 mm² vs 5.619 ± 0.918 cells/0.1 mm²). In addition, the number of goblet cells in the group that had been subjected to eye drop instillation with Diquas or Xiidra was increased about 1.1-fold as compared with the DS D10 control group (11.238 ± 0.436 cells/0.1 mm², 6.095 ± 1.082 cells/0.1 mm² vs 5.905 ± 1.190 cells/0.1 mm²). In particular, the number of goblet cells in the group that had been subjected 4 times to eye drop instillation with GBT-201 was restored to the level of goblet cells in the normal control group (Fig. 3).

### Experimental Example 3. Identification of increase in amount of mucin secreted following eye drop instillation with thymosin beta 4

In order to identify whether thymosin beta 4 results in an increase in amount of mucin secreted in the conjunctiva, the eyeballs of the mice sacrificed in Experimental Example 1 were collected and assessed through Alcian blue staining.

Specifically, the eyeballs of the mice sacrificed in Experimental Example 1 were collected, and the eyes and adnexa, which had been fixed in formalin for 3 days, were cut into 6 µm sections using a microtome. The sections of eyes and adnexa cut in 6 µm thickness were stained using Alcian Blue pH 2.5 Stain Kit (Abcam Inc, Cambridge, MA). If necessary, each of the sections was deparaffinized and hydrated with distilled water. The hydrated section was washed with distilled water, oxidized by being reacted with an acetic acid solution for 3 minutes, and then washed with distilled water. The oxidized section was reacted for 15 minutes in an Alcian blue solution so that mucin is stained blue.

Thereafter, for counter-staining, the section stained with Alcian blue was washed twice with distilled water, and then counter-stained using a Safranin O solution. Thereafter, the section was subjected to dehydration and clarification processes. Mounting thereof was performed using a mounting medium. For the mounted slide, photographing and tissue analysis were performed with a virtual microscope (NanoZoomer 2.0 RS, Hamamatsu Photonics K.K., Japan).

As a result, when the normal control group and the DS D10 control group were compared, it was visually identified that the amount of mucin secreted was decreased in the DS D10 control group as compared with the normal control group. On the other hand, as to the group that had been subjected to eye drop instillation with GBT-201, Diquas, or Xiidra, it was visually identified that the amount of mucin secreted was increased as compared with the DS D10 control group (Fig. 4).

Specifically, when the stained areas were quantified, the following results were observed. The amount of mucin secreted in the DS D10 control group was decreased by about 65.6% as compared with the amount of mucin secreted in the normal control group (14.952 ± 2.463 cells/0.1 mm² vs 5.143 ± 0.857 cells/0.1 mm²). On the other hand, the amount of mucin secreted in the GBT-201 group was increased about 2.6-fold as compared with the amount of mucin secreted in the DS D10 control group (12.095 ± 1.438 cells/0.1 mm², 13.143 ± 1.030 cells/0.1 mm² vs 5.143 ± 0.857 cells/0.1 mm²). In addition, the amount of mucin secreted in the group that had been subjected to eye drop instillation with Diquas or Xiidra was increased about 1.3-fold or about 1.5-fold, respectively, as compared with the DS D10 control group (6.571 ± 0.857 cells/0.1 mm², 7.714 ± 1.714 cells/0.1 mm² vs 5.143 ± 0.857 cells/0.1 mm²). In particular, the amount of mucin secreted in the two groups that had been subjected to eye drop instillation with GBT-201 was restored to the level of mucin secreted in the normal control group (Fig. 5).

### Experimental Example 4. Identification of increase in expression levels of Muc5AC, Muc1, Muc4, and Mucl6 following eye drop instillation with thymosin beta 4

In order to identify whether thymosin beta 4 results in an increase in expression levels of Muc5AC, Muc1, Muc4, and Muc16 in the conjunctiva, the eyeballs of the mice sacrificed in Experimental Example 1 were collected and stained with immunofluorescence.

Specifically, the eyeballs of the mice sacrificed in Experimental Example 1 were collected, and the eyes and adnexa, which had been fixed in formalin for 3 days, were cut into 6 µm sections using a microtome. Each of the corneal or conjunctival sections of eyes cut in 6 µm thickness was rehydrated with PBS, and then immersed in 0.3% Triton X-100 solution for 20 minutes. Thereafter, the section was washed with PBS three times, and then immersed in 3% bovine serum albumin (BSA) solution for 1 hour to prevent non-specific staining. Thereafter, treatment with anti-Mucl antibody (1:250, Abcam Inc, Cambridge, MA), anti-Muc4 antibody (1:250, Bioss Inc, Woburn, MA), anti-Muc5AC antibody (1:250, Thermo Fisher Scientific Inc., Waltham, MA), and anti-Mucl6 antibody (1:250, Abbiotec Inc, San Diego, CA) was performed and reaction was allowed to proceed overnight at a temperature of 4°C. The next day, the section was washed with PBS three times. Treatment with Alexa FluorTM 488 donkey anti-mouse IgG antibody (1:500; Thermo Fisher Scientific Inc, Waltham, MA) or Alexa FluorTM 555 donkey anti-rabbit IgG antibody (1:500; Thermo Fisher Scientific Inc, Waltham, MA) was performed and reaction was allowed to proceed at room temperature for 1 hour. Thereafter, the section was washed with PBS three times, and then mounting thereof was performed using a mounting medium containing DAPI. The mounted slide was photographed using a fluorescence microscope (Leica DM2500, Leica Microsystems GmbH, Wetzlar, Germany).

As a result, as illustrated in Figs. 6 and 7, Muc1, Muc4, and Muc16 were stained red in the surface layer of the corneal and conjunctival epithelium. When the normal control group and the DS D10 control group were compared, it was visually identified that the expression levels of Muc1, Muc4, and Muc16 were decreased in the DS D10 control group as compared with the normal control group. On the other hand, as to the group that had been subjected to eye drop instillation with GBT-201, it was visually identified that the expression levels of Muc1, Muc4, and Muc16 were remarkably increased as compared with the DS D10 control group.

In addition, as illustrated in Figs. 6 and 7, Muc5AC was stained green in the cornea and conjunctiva. When the normal control group and the DS D10 control group were compared, it was visually identified that the expression level of Muc5AC was decreased in the DS D10 control group as compared with the normal control group. On the other hand, as to the group that had been subjected to eye drop instillation with GBT-201 or Diquas, it was visually identified that the expression level of Muc5AC was increased as compared with the DS D10 control group. In particular, the expression level of GBT-201 was remarkably increased in the group that had been subjected to eye drop instillation with GBT-201.

## Claims

1. A composition for promoting goblet cell proliferation or mucin secretion, comprising as an active ingredient thymosin beta 4, thymosin beta 4 isoforms, or analogs or derivatives thereof.

2. The composition of claim 1, wherein the thymosin beta 4 has the amino acid sequence represented by SEQ ID NO: 1.

3. The composition of claim 1, wherein derivatives of thymosin beta 4 have a mutated N-terminus or C-terminus of thymosin beta 4.

4. The composition of claim 1, wherein the concentration of the thymosin beta 4, or isoforms or derivatives thereof is 0.02% (w/v) to 0.5% (w/v).

5. The composition of claim 1, further comprising acetic acid, citric acid, salts thereof, or hydrates of the salts.

6. The composition of claim 1, further comprising sodium chloride, potassium chloride, calcium chloride dihydrate, and magnesium chloride hexahydrate.

7. A pharmaceutical composition for preventing or treating goblet cell-related or mucin-related diseases, comprising as an active ingredient thymosin beta 4, thymosin beta 4 isoforms, or analogs or derivatives thereof.

8. The pharmaceutical composition of claim 7, wherein the thymosin beta 4 has the amino acid sequence represented by SEQ ID NO: 1.

9. The pharmaceutical composition of claim 7, wherein derivatives of thymosin beta 4 have a mutated N-terminus or C-terminus of thymosin beta 4.

10. The pharmaceutical composition of claim 7, wherein the concentration of the thymosin beta 4, or isoforms or derivatives thereof is 0.02% (w/v) to 0.5% (w/v).

11. The pharmaceutical composition of claim 7, further comprising acetic acid, citric acid, salts thereof, or hydrates of the salts.

12. The pharmaceutical composition of claim 7, further comprising sodium chloride, potassium chloride, calcium chloride dihydrate, and magnesium chloride hexahydrate.

13. The pharmaceutical composition of claim 7, wherein the mucin-related diseases are gastritis, gastric ulcer, enteritis, ulcerative colitis, or dry eye.

14. A method of treating goblet cell-related or mucin-related diseases, comprising:
administering, to a subject, the pharmaceutical composition of any one of claims 7 to 13.

15. The method of claim 14, wherein the goblet cell-related or mucin-related diseases are gastritis, gastric ulcer, enteritis, ulcerative colitis, or dry eye.

16. A use of the composition of claim 1, for promoting goblet cell proliferation or mucin secretion.

17. A use of the composition of claim 1, for manufacturing of a medicament for promoting goblet cell proliferation or mucin secretion.

18. A use of the composition of claim 7, for preventing or treating goblet cell-related or mucin-related diseases.

19. A use of the composition of claim 7 for manufacturing of a medicament for preventing or treating goblet cell-related or mucin-related diseases.
